# EUROPEAN PATENT APPLICATION

(11) **EP 4 299 727 A1**
(43) Date of publication of application: **03.01.2024**
(21) Application number: 21928188.8
(22) Date of filing: 15.06.2021
(51) Int. Cl.: C12N 7/00, A23K 10/16, A23K 20/195, A01N 63/40, A61K 35/76, A61P 31/04, A61P 31/02, C02F 1/68, C11D 3/38

(54) **NOVEL BACTERIOPHAGE HAVING CLOSTRIDIUM PERFRINGENS-SPECIFIC BACTERICIDAL EFFECT AND ANTIBACTERIAL COMPOSITION COMPRISING SAME**

(30) Priority: 25.02.2021 KR 20210025977
(71) Applicant: CJ Cheiljedang Corporation, Seoul 04560 (KR)
(72) Inventor: JEON, Jong Soo, Seoul 04560 (KR); KIM, Ji Eun, Seoul 04560 (KR); MOON, Jun Ok, Seoul 04560 (KR); CHAE, Jong Pyo, Seoul 04560 (KR); KIM, Yu Jin, Seoul 04560 (KR); LEE, Seung Eun, Seoul 04560 (KR)
(74) Representative: Bates, Philip Ian
(86) International application number: PCT/KR2021/007483
(87) International publication number: WO 2022/181893

(57) **Abstract**

The present application relates to a novel bacteriophage having Clostridium perfringens-specific bactericidal effect and an antibacterial composition comprising same, the novel bacteriophage CJ_CP_20-29 having Clostridium perfringens-specific bactericidal effect, and being superbly acid and heat resistant to allow wide use in antibiotics, feed and additives therefor, beverages and additives therefor, disinfectants, detergents, and the like to prevent or treat infectious diseases caused by Clostridium perfringens.

## Description

### [TECHNICAL FIELD]

The present application relates to a novel bacteriophage having specific bactericidal activity against *Clostridium perfringens* and an antibacterial composition comprising the same.

### [BACKGROUND ART]

*Clostridium perfringens* is a gram-positive large obligate anaerobic bacillus, and is known as a bacterium which has no flagella and forms spores. *Clostridium perfringens* is a bacterium which causes diarrhea and the like for animals, particularly, livestock including chickens, pigs and the like, and is recognized as one of important and fatal pathogens in livestock industry as much as poultry typhus caused by *Salmonella.*

Currently, necrotic enteritis caused by *Clostridium perfringens* infection, one of the most frequently occurring diseases in the poultry and hog industries, is a disease which causes a symptom that causes severe necrotic lesions in the lower small intestine of chickens or pigs or the like and excretes bloody diarrhea and the like as a major symptom. Such necrotic enteritis is a disease which becomes a problem significantly in the livestock industry, as it causes dehydration symptom, intermittent diarrhea symptom, and the like in infected animals depending on the severity of occurrence, and gradually weakens the animal's body, and causes growth failure, and the like. Furthermore, since *Clostridium perfringens,* which causes necrotic enteritis, is easily transmitted through animal feces, it is easily transmitted between animals in a common breeding space by oral infection and the like through soil or contaminated feeds, and in particular, the incidence rate of young livestock is high, so it becomes more problematic.

In the field such as livestock industry and the like, in order to prevent and treat bacterial infection of livestock, various antibiotics have been used, but as problems of antibiotic abuse of resistance, or problems of possibility of remaining in an animal body have been emerged, many restrictions are set on administration of an antibiotic worldwide at present.

Accordingly, in recent years, since bacteriophages which are bacteria-specific viruses that infect specific bacteria and inhibit growth of bacteria have stronger host specificity than antibiotics, they have been researched as alternatives of new antibiotics, but number thereof is absolutely insufficient so far. Therefore, in order to prevent and treat infectious disease caused by *Clostridium perfringens,* which becomes an important problem in the livestock industry including hog and poultry, development of a new bacteriophage, which has high industrial applicability as it has a wide spectrum of bacteriolysis and has excellent acid resistance and heat resistance, has been continuously demanded.

### [Prior art]

### [Patent document]

(Patent document 1) U.S. Patent US 6,942,858 B1

### [DISCLOSURE]

### [TECHNICAL PROBLEM]

An object of the present application is to provide bacteriophage CJ_CP_20-29 which has specific bactericidal activity against *Clostridium perfringens,* and is deposited under Accession number KCCM12935P, a composition for prevention or treatment of infectious disease caused by *Clostridium perfringens,* an antibiotic, a feed additive, a drinking water additive, a disinfectant, or a detergent comprising the same as an active ingredient, or a feed comprising the feed additive, or drinking water comprising the drinking water additive.

Another object of the present invention is to provide a method for prevention or treatment of infectious disease caused by *Clostridium perfringens* using the bacteriophage CJ_CP_20-29 or the composition for prevention or treatment of infectious disease caused by *Clostridium perfringens* comprising the same as an active ingredient.

Other object of the present invention is to provide a use for preparation of a prophylactic agent or a therapeutic agent of infectious disease caused by *Clostridium perfringens* of the bacteriophage CJ_CP_20-29 or the composition for prevention or treatment of infectious disease caused by *Clostridium perfringens* comprising the same as an active ingredient.

### [TECHNICAL SOLUTION]

Each description and embodiment disclosed in the present invention may be also applied to each other description and embodiment. In other words, all combinations of various elements disclosed in the present application fall within the scope of the present application. In addition, the scope of the present application is not to be construed as being limited by the specific description described below. Furthermore, those skilled in the art can recognize or confirm many equivalents to specific aspects described in the present application using only a common experiment. In addition, these equivalents are intended to be included in the present application.

One aspect provides bacteriophage CJ_CP_20-29 which has specific bactericidal activity against *Clostridium perfringens,* and is deposited under Accession number KCCM12935P.

The term used herein, *"Clostridium perfringens* (CP)" is a gram-positive anaerobic bacillus, and corresponds to a causative bacterium that causes infectious disease in an animal such as chickens, pigs, and the like.

The term used herein, "bacteriophage" is a bacteria-specific virus which infects a specific bacterium and inhibits growth of the corresponding bacterium, and means a virus comprising single or double-stranded DNA or RNA as a genetic material.

Another aspect provides a composition for prevention or treatment of infectious disease caused by *Clostridium perfringens* comprising the bacteriophage CJ_CP_20-29 as an active ingredient.

The bacteriophage CJ_CP_20-29 is as described above.

The bacteriophage CJ_CP_20-29 has specific bactericidal activity against *Clostridium perfringens,* so it can prevent infectious disease caused by *Clostridium perfringens* by killing *Clostridium perfringens* entering the subject, and inhibiting infection thereby, when administered before occurrence of infectious disease caused by *Clostridium perfringens* into a subject. In addition, when the bacteriophage CJ_CP_20-29 is administered into a subject in which infectious disease caused by *Clostridium perfringens* is developed, by killing *Clostridium perfringens* present in the subject, it can alleviate or extinct symptoms shown thereby, and thus, it can treat infectious disease caused by *Clostridium perfringens.*

The term used herein, "prevention" may mean all behaviors which inhibit or delay occurrence of disease in a subject by administration of a bacteriophage or a composition, and the term, "treatment" may mean all behaviors which improve, alleviate, or extinct symptoms of disease in a subject by administration of a bacteriophage or a composition.

The infectious disease caused by enterotoxigenic *Clostridium perfringens* may be for example, necrotic enteritis, but not limited thereto.

The term used herein, "necrotic enteritis" is one of infectious diseases caused by *Clostridium perfringens,* and corresponds to a bacterial disease that frequently occurs in livestock such as broilers and the like and causes considerable damage. Symptoms of necrotic enteritis appear as *Clostridium perfringens* excessively proliferates in the small intestine, and cause necrosis of digestive organ mucosa and sudden diarrhea and the like. For example, for hog, in case of peracute necrotic enteritis, death is caused in 1 day to 2 days after occurrence, and in case of acute necrotic enteritis, bloody diarrhea is excreted and in 2 days to 3 days, death is caused. In addition, in case of subacute necrotic enteritis, diarrhea (without bloody stools) lasts 5 days to 7 days, and weakness and dehydration are caused, and in case of chronic necrotic enteritis, intermittent diarrhea may be caused and growth failure may be caused.

The composition for prevention or treatment of infectious disease caused by *Clostridium perfringens* may comprise the bacteriophage CJ_CP_20-29 at 1 X 10 ² to 1 X 10 ¹² PFU/mL or 1 X 10 ⁵ to 1 X 10 ¹⁰ PFU/g.

The composition for prevention or treatment of infectious disease caused by *Clostridium perfringens* may further comprise a pharmaceutically acceptable carrier, and it may be provided as a food, medicine, feed additive or drinking water additive or the like as formulated with the carrier.

The term used herein, "pharmaceutically acceptable carrier" may mean a carrier or diluent which does not stimulate an organism and does not inhibit biological activity and characteristics of an administered compound.

The kind of the carrier to be comprised in the composition is not particularly limited, and any pharmaceutically acceptable carrier commonly used in the art may be used. Non-restrictive examples of the carrier may include saline solution, sterilized water, Ringer's solution, buffered saline solution, albumin injection solution, dextrose solution, malto-dextrin solution, glycerol and the like. They may be used alone or two or more kinds may be mixed and used, and if needed, it may be formulated as a formulation for injection such as solution, suspension, emulsion and the like, pill, capsule, granule or tablet by additionally adding a diluent, a dispersing agent, a binder and a lubricant.

The composition for prevention or treatment of infectious disease caused by *Clostridium perfringens* may be administered by oral administration or parenteral administration, and a method for applying or spraying to a disease area may be used. In case of parenteral administration, it may be administered using intravenous administration, intraperitoneal administration, intramuscular administration, subcutaneous administration or topical administration. An amount of applying, spraying and administering of the composition varies according to factors such as formulation method, administration method, target animal and patient's age, body weight, gender, degree of disease symptoms, food, administration time, administration route, excretion rate and reaction sensitivity, and a commonly skilled doctor or veterinarian may easily determine and prescribe an effective dose for desired treatment.

As a formulation for oral administration of the composition for prevention or treatment of infectious disease caused by *Clostridium perfringens,* for example, it may be formulated as a tablet, troche, lozenge, aqueous or oil suspension, prepared powder or granule, emulsion, hard or soft capsule, syrup or elixir. In order to formulate it as a formulation such as a tablet or capsule or the like, it may comprise a binder such as lactose, saccharose, sorbitol, mannitol, starch, amylopectin, cellulose or gelatin, an excipient such as dicalcium phosphate, a disintegrant such as corn starch or sweat potato starch, or a lubricant such as magnesium stearate, calcium stearate, sodium stearyl fumarate, or polyethylene glycol wax, and in case of the capsule formulation, a liquid carrier such as fat oil may be further contained in addition to the afore-mentioned materials.

As a formulation for parenteral administration of the composition for prevention or treatment of infectious disease caused by *Clostridium perfringens,* it may be formulated in a form for injection such as subcutaneous injection, intravenous injection or intramuscular injection or the like, or for spray such as aerosol allowing inhalation through a suppository injection method or a respirator. In order to formulate it in a formulation for injection, the composition of the present invention may be prepared as a solution or suspension by mixing with a stabilizer or buffer in water, and this may be formulated for unit administration of an ample or vial. When it is formulated as a spray such as aerosol and the like, a propellant or the like may be blended with an additive so that a water-dispersed concentrate or wet powder.

The composition for prevention or treatment of infectious disease caused by *Clostridium perfringens* may comprise a preservative, stabilizer, wetting agent or emulsifier, cryoprotectant, or excipient, or the like.

The preservative, stabilizer, or excipient may be comprised in an effective dose sufficient for reducing deterioration of the composition in the composition.

The term used herein, "deterioration" may comprise a decrease in the number of bacteria, a decrease in activity, of the bacteriophage CJ_CP_20-29 comprised in a composition and the like, or a combination thereof.

For example, as the composition comprises an effective dose of preservative, stabilizer or excipient, sufficient for reducing deterioration of the composition, differently from a bacteriophage present in nature, the bacteriophage CJ_CP_20-29 may survive or maintain activity for a longer period of time in the composition.

As the preservative, stabilizer, or excipient, those commonly used in the art may be used without limitation, as long as they can reduce deterioration of the composition. For example, the stabilizer may be any one or more selected from the group consisting of alginate, sodium alginate, casein, sodium casein, cellulose, methyl cellulose, carboxymethylcellulose, sodium carboxymethylcellulose, pectin, gelatin, Arabic gum, xanthan gum, dextran, cyclodextrin and starch.

The cryoprotectant may be comprised in an effective dose sufficient for reducing deterioration of the composition in the composition, when the composition is freeze-dried. For example, differently from a bacteriophage present in nature which has lowered activity or cannot survive in the freeze-dried composition, the bacteriophage activity may be maintained or the bacteriophage may survive even in the freeze-dried composition, as the composition comprises an effective dose of cryoprotectant sufficient for reducing deterioration of the composition in a freeze-dried state.

As the cryoprotectant, those commonly used in the art may be used without limitation, as long as it is to reduce deterioration of the composition in a freeze-dried state. For example, the cryoprotectant may be any one or more selected from the group consisting of glycerol, ethylene glycol, propylene glycol, dimethyl sulfoxide (DMSO), glucose, trehalose, maltodextrin, dextrin, skim milk and starch.

Other aspect provides an antibiotic comprising the bacteriophage CJ_CP_20-29 as an active ingredient.

The bacteriophage CJ_CP_20-29 is as described above.

The term used herein, "antibiotic" may mean an agent capable of killing bacteria or inhibiting growth of bacteria, and may collectively call preservatives, bactericides, and antimicrobials.

The antibiotic comprising the bacteriophage CJ_CP_20-29 as an active ingredient has very high specificity against *Clostridium perfringens* compared to conventional antibiotics, so it cannot kill beneficial bacteria and can kill specific pathogenic bacteria only, and it does not induce drug resistance, so it can exhibit an effect of extending a product lifespan compared to conventional antibiotics.

The antibiotic may be prepared by a method for preparation commonly used in the art.

The antibiotic may comprise an effective dose of additive sufficient for reducing deterioration of the antibiotic, and the additive may include a preservative, stabilizer, excipient, or cryoprotectant, or the like. As the antibiotic comprises the additive, differently from a bacteriophage present in nature, the bacteriophage CJ_CP_20-29 may survive or maintain activity for a longer period of time in the antibiotic. As the preservative, stabilizer, excipient or cryoprotectant, those commonly used in the art may be used without limitation, as long as it can reduce deterioration of the antibiotic.

Other aspect provides a feed additive comprising the bacteriophage CJ_CP_20-29 as an active ingredient. Other aspect provides a feed comprising the feed additive. Other aspect provides a drinking water additive comprising the bacteriophage CJ_CP_20-29 as an active ingredient. Other aspect provides drinking water comprising the drinking water additive.

The bacteriophage CJ_CP_20-29 is as described above.

The feed additive or drinking water additive may be used by a method of mixing in a feed or drinking water as prepared in a form of a composition comprising the bacteriophage CJ_CP_20-29, or directly adding the bacteriophage CJ_CP_20-29 during preparation of the feed or drinking water.

The bacteriophage CJ_CP_20-29 comprised in the feed additive or drinking water additive may be in a liquid state or in a dried solid state, and specifically, it may be in a dried powder form. The method of drying of the bacteriophage CJ_CP_20-29 may be air drying, natural drying, spray drying or freeze-drying, but not limited thereto. The bacteriophage CJ_CP_20-29 may be mixed in a dried powder form in an amount of 0.05 to 10 % by weight, specifically, 0.1 to 2 % by weight of the weight of the feed additive or drinking water additive.

The feed additive or drinking water additive comprising the bacteriophage CJ_CP_20-29 as an active ingredient may further comprise other additives if needed. Non-restrictive examples of the usable additives include a binder, emulsifier, preservative and the like added to prevent deterioration of a feed or drinking water; and an amino acid agent, vitamin agent, enzyme agent, probiotic, flavoring agent, non-protein nitrogen compound (NPN), silicate agent, buffer, coloring agent, extractant or oligosaccharide or the like, and they may be added along or two or more kinds may be added together.

Raw materials other than the bacteriophage CJ_CP_20-29 comprised in the feed or drinking water may be used without limitation as those commonly used in the art. Non-restrictive examples of the feed may include vegetable feeds such as grains, root fruits, food processing by-products, algae, fibers, pharmaceutical by-products, oils and fats, starches, meal or grain by-products, or the like; and animal feeds such as proteins, inorganic materials, oils and fats, minerals, oils and fats, monocyte proteins, zooplanktons or food, or the like. They may be used alone or used by mixing 2 or more kinds.

The feed additive may be comprised in an amount of 0.05 to 10 parts by weight, for example, 0.1 to 2 parts by weight, based on 100 parts by weight of feed. The drinking water additive may be comprised in an amount of 0.0001 to 0.01 parts by weight, for example, 0.001 to 0.005 parts by weight, based on 100 parts by weight of drinking water.

The feed additive, feed, drinking water additive or drinking water may be prepared by a method for preparation commonly used in the art.

The feed additive, feed, drinking water additive or drinking water may comprise an effective dose of an additive sufficient to reduce deterioration thereof, and the additive may be a preservative, stabilizer, excipient or cryoprotectant, or the like. The feed additive, feed, drinking water additive or drinking water may survive or maintain activity for a longer period than the bacteriophage CJ_CP_20-29 in the feed additive, feed, drinking water additive or drinking water, differently from the bacteriophage present in nature, by comprising the additive. As long as the preservative, stabilizer, excipient, or cryoprotectant can reduce deterioration of the feed additive, feed, drinking water additive or drinking water, those commonly used in the art may be used without limitation.

Other aspect provides a disinfectant comprising the bacteriophage CJ_CP_20-29 as an active ingredient. Other aspect provides a detergent comprising the bacteriophage CJ_CP_20-29 as an active ingredient.

The bacteriophage CJ_CP_20-29 is as described above.

The formulation of the disinfectant or detergent is not particularly limited, and it may be prepared into a formulation commonly known in the art and used. The disinfectant or detergent may be prepared by a method for preparation commonly used in the art.

The disinfectant may be sprayed to remove *Clostridium perfringens,* and may be sprayed on animal activity areas, slaughterhouses, dead areas, cooking places or cooking facilities or the like, but not limited thereto.

The detergent may be used for a use of washing a skin surface or each body part or the like of an animal which is exposed to or likely to be exposed to *Clostridium perfringens,* but not limited thereto.

The disinfectant or detergent may comprise an effective dose of an additive sufficient to reduce deterioration thereof, and the additive may be a preservative, stabilizer, excipient, or cryoprotectant, or the like. The disinfectant or detergent may survive or maintain activity for a longer period than the bacteriophage CJ_CP_20-29 in the disinfectant or detergent, differently from the bacteriophage present in nature, by comprising the additive. As long as the preservative, stabilizer, excipient, or cryoprotectant can reduce deterioration of the disinfectant or detergent, those commonly used in the art may be used without limitation.

Other aspect provides a method for prevention or treatment of infectious disease caused by *Clostridium perfringens,* comprising administering the bacteriophage CJ_CP_20-29, or a composition for prevention or treatment of infectious disease caused by *Clostridium perfringens* comprising this as an active ingredient into an animal except for humans.

The bacteriophage CJ_CP_20-29, composition for prevention or treatment of infectious disease caused by *Clostridium perfringens* comprising this as an active ingredient, and infectious disease caused by *Clostridium perfringens* are as described above. Specifically, the infectious disease caused by *Clostridium perfringens* may be for example, necrotic enteritis.

The method for prevention or treatment specifically comprises administering a pharmaceutically effective dose of the bacteriophage CJ_CP_20-29, or a composition comprising this as an active ingredient into an animal except for humans, which are infected or at risk of being infected with *Clostridium perfringens.* The bacteriophage CJ_CP_20-29, or a composition comprising this as an active ingredient may be administered into an animal in a form of pharmaceutical formulations, or may be administered by a method for mixing in a form of feed additives or drinking water additives into a feed or drinking water of an animal and feeding this.

The administration route of the bacteriophage CJ_CP_20-29 or composition comprising the same as an active ingredient may be various oral or parenteral routes as long as it can reach target tissue, and specifically, it may be administered by a common method through oral, rectal, local, intravenous, intraperitoneal, intramuscular, intraarterial, transdermal, intranasal, inhalation and the like.

A suitable daily total amount used of the bacteriophage CJ_CP_20-29 or composition comprising the same as an active ingredient may be determined within the scope of correct medical judgment by a treatment doctor, and this is an obvious matter to those skilled in the art.

A specific pharmaceutically effective dose of the bacteriophage CJ_CP_20-29 or composition comprising the same as an active ingredient for a specific animal, may be determined in consideration of the type and degree of a response to be achieved, the age, body weight, general health condition, gender or diet of the corresponding subject, as well as, the administration time and administration route of the bacteriophage CJ_CP_20-29 or the composition comprising the same as an active ingredient, and the secretion rate of the composition, treatment period and the like, and it may vary depending on various factors including drugs and other components of the composition used simultaneously or at different times together and similar factors well known in the medical field.

As the animal except for humans, any animal which can be infected by *Clostridium perfringens* is not particularly limited. For example, the animal may be a bird or mammal, and specifically, it may include chickens, ducks, pigs, and the like, but not limited thereto.

Other aspect provides a use for preparation of a prophylactic agent or a therapeutic agent of infectious disease caused by *Clostridium perfringens* of a composition for prevention or treatment of infectious disease caused by *Clostridium perfringens* of the bacteriophage CJ_CP_20-29, or a composition for prevention or treatment of infectious disease caused by *Clostridium perfringens* comprising the same as an active ingredient.

The bacteriophage CJ_CP_20-29, composition for prevention or treatment of infectious disease caused by *Clostridium perfringens* comprising the same as an active ingredient, and infectious disease caused by *Clostridium perfringens* are as described above. Specifically, the infectious disease caused by *Clostridium perfringens* may be for example, necrotic enteritis.

### [ADVANTAGEOUS EFFECTS]

The novel bacteriophage CJ_CP_20-29 has an effect of specifically killing *Clostridium perfringens,* and has excellent acid resistance and heat resistance, so it can be widely utilized for an antibiotic, a feed additive, a drinking water additive, a feed, drinking water, a disinfectant or a detergent, or the like as a use for prevention or treatment of infectious disease caused by *Clostridium perfringens.*

### [BRIEF DESCRIPTION OF THE DRAWINGS]

FIG. 1 is an electron microscope photograph of the novel bacteriophage CJ_CP_20-29.
FIG. 2 is a result graph of confirming pH stability of the novel bacteriophage CJ_CP_20-29.
FIG. 3 is a result graph of confirming stability at 60°C of the novel bacteriophage CJ_CP_20-29.

### [MODE FOR INVENTION]

Hereinafter, the present invention will be described in more detail by examples. However, these examples are intended to illustratively describe one or more embodiments, but the scope of the present invention is not limited by these examples.

### Example 1. Separation of bacteriophage having bactericidal activity against Clostridium perfringens (CP)

### Example 1-1. Preparation of fecal sample pretreatment solution

Fecal samples of hog, poultry and cattle were collected from farms in Seoul, Gyeonggi-do, Chungcheong-do and Gyeongsang-do areas of Korea, and 20g of each sample was diluted in PBS of 80 mL and centrifuged at 10,000 rpm for 15 minutes. The supernatant was filtered with a 0.2 µm filter, and then 10%(w/v) sodium chloride aqueous solution was added to the filtrate, and stored at 4°C for 12 hours. After that, 10%(w/v) polyethylene glycol 8000 (Sigma-Aldrich, Cat. No. P2139) was added thereto, and stored at 4°C for 12 hours, and then for concentration, it was centrifuged at 15,000 rpm for 1 hour to remove the supernatant. The precipitate was dissolved in 10 mL of SM buffer (5.8 g/L sodium chloride, 2 g/L MgSO ₄·7H ₂O, 0.05M Tris-Cl (pH 7.5)), and then filtered with a 0.2 µm filter, and the filtrate was stored at 4°C.

### Example 1-2. Production of bacteriophage concentrated solution

CP strains separated from fecal samples collected from livestock farms of Seoul, Gyeonggi-do, Chungcheong-do and Gyeongsang-do areas of Korea and CP strains sold from National Veterinary Research and Quarantine Service were inoculated into a BHI (Brain Heart Infusion) medium of 2mL, and standing cultured at 42°C under an anaerobic condition for 18 hours, and then, the CP strain cultured solution of 1mL and the fecal sample pretreatment solution obtained from Example 1-1 of 1 mL were inoculated into the BHI medium of 50 mL, and mixed and standing cultured at 42°C for 18 hours. After the mixed cultured solution was centrifuged at 6,000 rpm for 20 minutes, the supernatant was filtered with a 0.2 µm filter, and 10%(w/v) polyethylene glycol 8000 was added thereto, and stored at 4°C for 12 hours. After that, for concentration, it was centrifuged at 15,000 rpm for 1 hour to remove the supernatant, and the precipitate was dissolved in 1 mL of SM buffer, and it was filtered with a 0.2 µm filter, and the filtrate was stored at 4°C.

### Example 1-3. Screening and separation of bacteriophage

50 µL of the bacteriophage concentrated solution produced in Example 1-2 was mixed with 5 mL of 0.7%(w/v) agar (BD DIFCO, Cat. No. 44164) and 50 µL of the cultured solution in which the CP strain as same as used in Example 1-2 was cultured with shaking so that the absorbance (O.D.) was 2, and using a BHI plate medium with a diameter of 150 mm, double-layer agar plaque assay was performed. Plaques formed on soft agar were punched with a 200 µL tip, and added to 0.5 mL of SM buffer to elute. Double-layer agar plaque assay was repeated for a solution comprising the eluted bacteriophage until a single plaque in the same form was formed to separate a solution comprising a pure bacteriophage. The obtained solution comprising the bacteriophage was filtered with a 0.2 µm filter, and 10%(w/v) polyethylene glycol 8000 was added thereto, and stored at 4°C for 12 hours. After that, for concentration, it was centrifuged at 15,000 rpm for 1 hour to remove the supernatant, and the precipitate was dissolved in 1 mL of SM buffer, and it was filtered with a 0.2 µm filter, and the filtrate was stored at 4°C.

### Example 2. Whole genome sequencing (WGS) of separated bacteriophage

DNA was extracted from 1 mL of the pure separated bacteriophage concentrated solution in Example 1-3 using CsCl gradient method and a phage DNA separation kit (Norgen Biotek-Corp. Kit, Cat. No. 46800). Whole genome sequencing was performed by requesting to Macrogen Inc., and genes were combined using De novo assembly software (SPAdes 3.13.0), and Open reading frame (ORF) was performed using GeneMark.hmm and NCBI ORF finder. The function of each ORF was annotated using BLASTP (E values of <0.1) and PSI-BLAST (E value of <0.005) programs.

As a result, it was confirmed that the separated bacteriophage had the base sequence of SEQ ID NO: 1 with 51,669 bp, 72 ORF, G+C content 34.10%, and this showed the sequence identity of 97% with conventionally reported *Clostridium* phage CP3 (MF001357.1), but it was confirmed that there was no bacteriophage of which all fragments matched 100%, and it could be seen that the bacteriophage was a novel separated bacteriophage. Accordingly, the novel bacteriophage was named bacteriophage CJ_CP_20-29, and deposited to Korean Culture Center of Microorganisms, which is an international depository institution under Budapest Treaty on January 18, 2021, and was given Accession number KCCM12935P.

### Example 3. Morphology analysis of bacteriophage CJ_CP_20-29

In order to obtain a high purity of bacteriophage solution, CsCl gradient method was performed. Specifically, a CsCl solution dissolved in SM buffer of which density was 1.7, 1.5, 1.45 or 1.3 was prepared, and the CsCl solution was aliquoted in a 15 mL ultracentrifuge tube (Beckman Coulter, Cat. No. Z00901SCA) by 2 mL each so as to layer from high density to low density, and 2 mL of the bacteriophage CJ_CP_20-29 concentrated solution obtained in Example 1-3 was aliquoted at the top.

This was centrifuged at 4°C and 25,000 rpm for 2 hours, and then only the white bacteriophage layer formed in the tube was collected with a syringe (Satorius, Cat. No. 17822-K). After dropping 1 µL of the collected bacteriophage solution on carbon-coated copper grid, it was stained with 2% uranyl acetate for 15 seconds and the morphology was observed with an electron microscope (TEM, JEOL JEM-101, Tokyo, Japan).

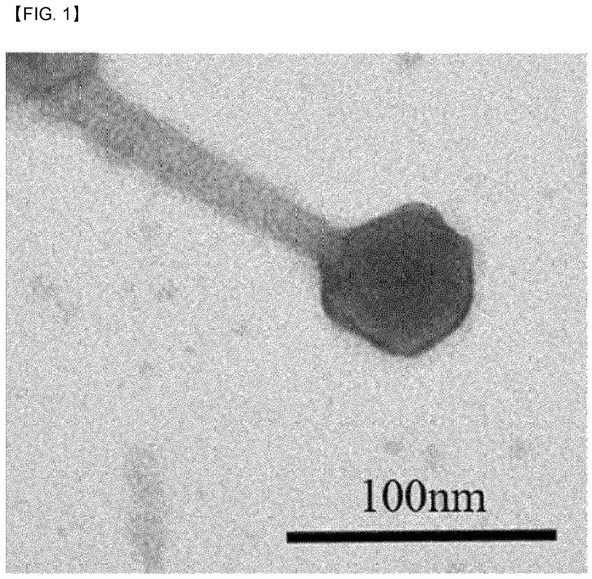

As a result, as shown in FIG. 1, it was observed that the bacteriophage CJ_CP_20-29 had a morphologically icosahedral head and a contractile tail with a length of about 100 nm, and thereby, it could be seen that it belonged to *Caudovirales* order, *Myoviridae* family.

### Example 4. Evaluation of pH stability of bacteriophage CJ_CP_20-29

In order to confirm that the bacteriophage CJ_CP_20-29 has stability in a broad pH range, a solution with pH 4, 7, 7.5 or 10 (pH 4: 0.2M sodium acetate solution; pH 7, 7.5: 0.2M sodium phosphate solution; and pH 10: 0.2M Tris(Tris-HCl) solution) was prepared. After 450 µL of the solution by each pH and 50 µL of the bacteriophage solution of 2 X 10 ¹⁰ PFU/mL were mixed and stood at 4°C for 2 hours, double-layer agar plaque assay was performed to evaluate titer increase and decrease.

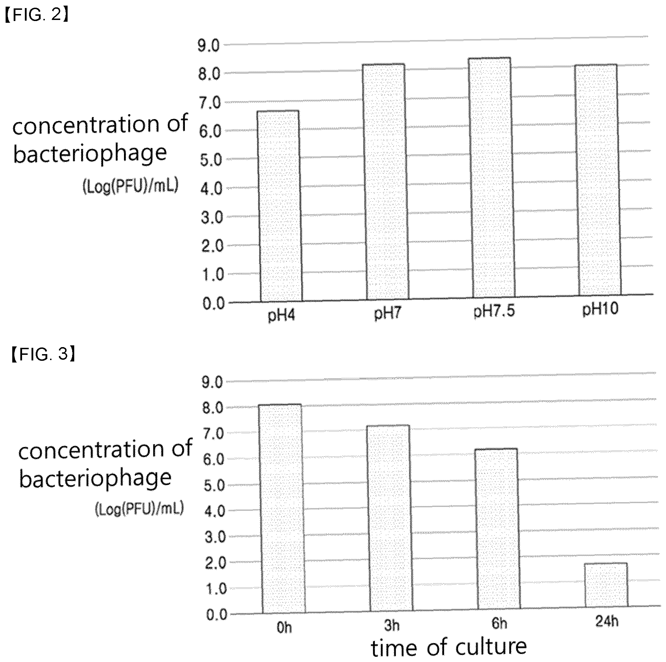

As a result, as shown in FIG. 2, the bacteriophage CJ_CP_20-29 was stable without losing activity in a range of pH 7 to 10, and even at pH 4, compared to the group at pH 7.5, the activity was reduced by only about 1.7 logs, so it could be seen that it was a bacteriophage having excellent acid resistance.

### Example 5. Evaluation of thermal stability of bacteriophage CJ_CP_20-29

In order to confirm whether the bacteriophage CJ_CP_20-29 had stability at a high temperature, after standing 500 µL of the bacteriophage solution of 2 X 10 ⁸ PFU/mL at 60°C for 0, 3, 6 or 24 hours, double-layer agar plaque assay was performed to evaluate titer increase and decrease.

As a result, as shown in FIG. 3, the bacteriophage CJ_CP_20-29 showed a decrease in activity of about 1.0 log compared to the control group exposed for 0 hour, when it is exposed at 60°C for 3 hours, and showed a decrease in activity of about 1.9 logs compared to the control group when exposed for 6 hours, and even when exposed for 24 hours, it showed a decrease in activity of about 6.4 logs compared to the control group, but still showed the activity. Therefore, it could be seen that the bacteriophage CJ_CP_20-29 was a bacteriophage having excellent heat resistance.

### Example 6. Evaluation of bacteriolysis spectrum of bacteriophage CJ_CP_20-29

In order to evaluate the bacteriolysis range of the bacteriophage CJ_CP_20-29, a total of 45 kinds of the CP strain from fecal samples collected from livestock farms of Seoul, Gyeonggi-do, Chungcheong-do and Gyeongsang-do areas of Korea and CP strain sold from National Veterinary Research and Quarantine Service were cultured in a BHI liquid medium, respectively, and then 50 µL of each strain culture was inoculated into 5 mL of 0.7% soft agar and poured in a petri dish and plated, and then stood for 5 minutes. After that, 10 µL of the bacteriophage CJ_CP_20-29 concentrated solution obtained in Example 1-3 was spotted on soft agar, and then standing cultured at 42°C for 18 hours. After completing the culture, the bacteriolysis range of the bacteriophage CJ_CP_20-29 was evaluated according to presence or absence of plaques formed on soft agar.

**[Table 1]**

| CP strain name | Presence or absence of plaque formation | CP strain name | Presence or absence of plaque formation |
|---|---|---|---|
| HLYS-1 | + | BCCP42-2 | ++ |
| HLYS-3 | ++ | BCCP43-1 | + |
| JSH-1 | ++ | BCCP47-2 | ++ |
| CP-KCCM 40947 | ++ | BCCP48-3 | ++ |
| KJW-2 | ++ | BCCP51-1-1 | ++ |
| CP-KJW-1 | ++ | BCCP52-2-8 | ++ |
| CP-JSH-1 | ++ | BCCP53-2-3 | ++ |
| CP-OYS-2 | ++ | BCCP54-3-8 | ++ |
| CP-BC-1 | ++ | BCCP55-3-1 | ++ |
| CP-BSW-4 | ++ | SBCCP429-2 | ++ |
| CP-HBM-2 | ++ | SBCCP321 | + |
| CP-HLYS | ++ | SBCCP343 | ++ |
| CP-KW-1 | ++ | SBCCP361 | ++ |
| CP-BS-1 | ++ | ELCCP6-1 | + |
| CP-HL-1 | ++ | OYS-2-1 | ++ |
| CP-LJN-1 | ++ | OYS-2-2 | ++ |
| BCCP17-1 | ++ | 1-1-2 | + |
| BCCP23-4 | ++ | 1-1 | ++ |
| BCCP37-2 | ++ | C3 | ++ |
| BCCP38-1 | ++ | CP-ATCC12921 | + |
| BCCP39-1 | ++ | CP-ATCC13124 | ++ |
| BCCP40-1 | ++ | CP-CCARM 18020 | ++ |
| BCCP41-3 | ++ | | |

| | | | |
|---|---|---|---|
| (++: Clean plaques are formed; +: Turbid plaques are formed) | | | |

As a result, as shown in Table 1, it was confirmed that plaques were formed in all the 45 kinds of the tested CP strains, so the bacteriophage CJ_CP_20-29 had bactericidal activity against widespread CP strains.

From the above description, those skilled in the art to which the present invention pertains will understand that the present invention may be embodied in other specific forms without changing its technical spirit or essential features. In this regard, the examples described above should be understood as illustrative not restrictive in all respects. The scope of the present invention should be construed as including all changed or modified forms derived from the meaning and scope of claims described below and equivalents thereof rather than the detailed description above.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
CJ_CP_20-29 | Accession number given by the
INTERNATIONAL DEPOSITARY AUTHORITY:
KCCM12935P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 18. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 18. 2021.

¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. Bacteriophage CJ_CP_20-29 which has specific bactericidal activity against *Clostridium perfringens* (CP), and is deposited under Accession number KCCM12935P.

2. A composition for prevention or treatment of infectious disease caused by *Clostridium perfringens* comprising the bacteriophage CJ_CP_20-29 of claim 1 as an active ingredient.

3. The composition according to claim 2, wherein the infectious disease caused by *Clostridium perfringens* is necrotic enteritis.

4. An antibiotic comprising the bacteriophage CJ_CP_20-29 of claim 1 as an active ingredient.

5. A feed additive comprising the bacteriophage CJ_CP_20-29 of claim 1 as an active ingredient.

6. A feed comprising the feed additive of claim 5.

7. A drinking water additive comprising the bacteriophage CJ_CP_20-29 of claim 1 as an active ingredient.

8. Drinking water comprising the drinking water additive of claim 7.

9. A disinfectant comprising the bacteriophage CJ_CP_20-29 of claim 1 as an active ingredient.

10. A detergent comprising the bacteriophage CJ_CP_20-29 of claim 1 as an active ingredient.

11. A method for prevention or treatment of infectious disease caused by *Clostridium perfringens,* comprising a step of administering the bacteriophage CJ_CP_20-29 of claim 1 or the composition of claim 2 into an animal except for humans.

12. The method according to claim 11, wherein the infectious disease caused by *Clostridium perfringens* is necrotic enteritis.
